(19) Europäisches Patentamt
European Patent Office
Office européen des brevets

(11) EP 0 640 073 B1

# (12) EUROPÄISCHE PATENTSCHRIFT

(45) Veröffentlichungstag und Bekanntmachung des Hinweises auf die Patenterteilung:
**17.04.1996 Patentblatt 1996/16**

(21) Anmeldenummer: **93909543.6**

(22) Anmeldetag: **05.05.1993**

(51) Int Cl.$^6$: **C07D 215/10**, C23C 18/00, C07D 213/20, C07D 217/10

(86) Internationale Anmeldenummer:
**PCT/EP93/01090**

(87) Internationale Veröffentlichungsnummer:
**WO 93/23377 (25.11.1993 Gazette 1993/28)**

## (54) VERFAHREN ZUR HERSTELLUNG VON N-ALLYLVERBINDUNGEN

PROCESS FOR PREPARING N-ALLYL COMPOUNDS

PROCEDE DE FABRICATION DE COMPOSES DE N-ALLYLE

(84) Benannte Vertragsstaaten:
**BE DE ES FR GB IT**

(30) Priorität: **16.05.1992 DE 4216314**

(43) Veröffentlichungstag der Anmeldung:
**01.03.1995 Patentblatt 1995/09**

(73) Patentinhaber: **BASF Aktiengesellschaft
D-67063 Ludwigshafen (DE)**

(72) Erfinder:
- **BURKHART, Bernd
D-6704 Mutterstadt (DE)**
- **OFTRING, Alfred
D-6702 Bad Duerkheim (DE)**
- **WIDDER, Rudi
D-6906 Leimen (DE)**
- **SCHROEDER, Ulrich
D-6710 Frankenthal (DE)**

(56) Entgegenhaltungen:
**DE-A- 1 952 356**
**JP-A-62 000 447**
**US-A- 4 029 658**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Aus der US-Patentschrift 4 029 658 ist schon ein Verfahren zur Herstellung von Allylpyridiniumchlorid bekannt, bei dem Pyridin mit Allylchlorid in Dimethylformamid umgesetzt wird.

Ferner ist aus der japanischen Patentveröffentlichung 62000447 die Umsetzung von Allylchlorid mit Pyridin im Autoklaven bekannt.

N-Allylverbindungen, insbesondere solche des Pyridins mit wasserlöslichen Anionen sind in der Regel hygroskopisch und neigen daher stark zum Verklumpen, was ihre Anwendbarkeit bei technischen Prozessen sehr erschwert. Es bestand daher der Wunsch, solche Verbindungen in leicht handhabbarer Form zur Verfügung zu haben.

Überraschenderweise wurde nun gefunden, daß wäßrige Lösungen eine solche Form darstellen und daß sich diese wäßrigen Lösungen in einfacher und kostengünstiger Weise herstellen lassen.

Die Erfindung betrifft nun das Herstellungsverfahren gemäß Anspruch 1.

Heterocyclen, die sich erfindungsgemäß mit den Verbindungen der Formel III umsetzen lassen, sind insbesondere Chinolin- und Isochinolinverbindungen, die noch durch $C_1$- bis $C_4$-Alkyl substituiert sein können, und vorzugsweise Pyridin, das gegebenenfalls noch Alkylreste tragen kann. Besonders bevorzugt ist Pyridin selbst.

Verbindungen der Formel III sind insbesondere Allyl- oder $\alpha$-, $\beta$-, $\gamma$-Methallylhalogenide, von denen die Chloride und Bromide bevorzugt sind; n in Formel I ist daher vorzugsweise 1. Es ist natürlich möglich, die Halogenidanionen gegen andere wasserlöslich machende Anionen nach an sich bekannten Methoden auszutauschen.

Vorzugsweise wird die erfindungsgemäße Reaktion in rein wäßrigem Medium vorgenommen, es ist aber natürlich auch möglich, ein wasserlösliches, organisches Lösungsmittel wie einen Alkohol, ein Glykol, Tetrahydrofuran, ein Amid oder ein Keton mitzuverwenden. Ebenfalls ist es möglich, die Umsetzung in Gegenwart eines Emulgators, insbesondere eines solchen des Öl-in-Wasser-Typs, durchzuführen. Ein derartiges wasserlösliches organisches Lösungsmittel und ein derartiger Emulgator können auch gleichzeitig bei der Umsetzung anwesend sein.

Weiterhin bevorzugt ist die Umsetzung der Verbindungen II und III in stöchiometrischen Mengen, wobei die Reihenfolge der Zugabe beliebig ist. Es kann also entweder die Verbindung der Formel II oder III zur III oder II enthaltenden wäßrigen Lösung oder es können die Verbindungen II und III zum Wasser zugegeben werden.

Zweckmäßigerweise wird die Umsetzung bei erhöhter Temperatur, beispielsweise kurz unter dem Siedepunkt des Reaktionsgemisches vorgenommen. Die Konzentrationen werden so gewählt, daß 20- bis 80 %ige, vorzugsweise 50- bis 80 %ige Lösungen der Verbindungen der Formel I entstehen.

Um Reste der nicht umgesetzten Verbindungen der Formeln II und III zu entfernen, kann man nach Beendigung der Umsetzung eine Wasserdampfdestillation anschließen oder ein inertes Gas durch das Reaktionsgemisch leiten. Inerte Gase sind z.B. Stickstoff, $CO_2$ oder auch Luft. Dabei ist darauf zu achten, daß pH-Werte von 4 bis 8, vorzugsweise 5 bis 6 eingehalten werden. Diese pH-Werte können z.B. durch Zugabe von Natronlauge eingehalten werden.

Einzelheiten der Herstellung und Reaktionsführung können den folgenden Beispielen entnommen werden, in denen sich Angaben über Teile und Prozente, sofern nicht anders vermerkt, auf das Gewicht beziehen.

Die erfindungsgemäß hergestellten Produkte eignen sich z.B. als Glanzbildner bei der Herstellung vernickelter Formteile durch galvanische Abscheidung von Nickel aus wäßrig-sauren Bädern.

Beispiel 1

Herstellung von Pyridiniumallylchlorid

Man legt 466,5 g Wasser vor. Dazu gibt man 229,5 g (3 mol) Allylchlorid und erhitzt auf 50°C. Danach werden unter gutem Rühren innerhalb einer Stunde 237,0 g (3,0 mol) Pyridin zugetropft. Nach weiteren 8 Stunden bei 50°C wird eine Wasserdampfdestillation bei 500 mbar/80°C durchgeführt. Während der Wasserdampfdestillation wird der pH-Wert mittels verd. NaOH auf 5 gehalten. Man destilliert solange bis im Sumpf der Pyridinwert kleiner als 0,1 Gew.% ist.
Die Ausbeute beträgt 90 %.

Beispiel 2

Herstellung von Pyridinium-$\beta$-methallylchlorid

Die Titelverbindung wurde analog zu Beispiel 1 aus Pyridin und $\beta$-Methallylchlorid in einer Ausbeute von 87 % hergestellt.

Beispiel 3

Herstellung von Pyridinium-γ-methallylchlorid

Die Titelverbindung wurde analog zu Beispiel 1 aus Pyridin und γ-Methallylchlorid in einer Ausbeute von 83 % hergestellt.

**Patentansprüche**

**1.** Verfahren zur Herstellung von N-Allylverbindungen der allgemeinen Formel I

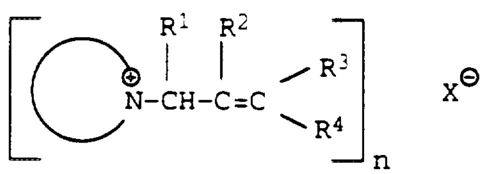

I,

in der 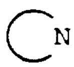 einen N-haltigen aromatischen Heterocyclus

| | |
|---|---|
| $R^1$, $R^3$ und $R^4$ | unabhängig voneinander Wasserstoff oder $C_1$- bis $C_4$-Alkyl, |
| $R^2$ | Wasserstoff oder Methyl, |
| n | 1, 2, 3 oder 4 und |
| $X^\ominus$ | ein wasserlöslich machendes Anion |

bedeuten, dadurch gekennzeichnet, daß man Verbindungen der Formel II

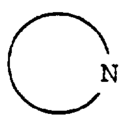

II,

mit Verbindungen der Formel III

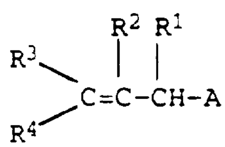

III,

in der A einen als Anion abspaltbaren Rest bedeutet, in wäßrigem Medium umsetzt.

**2.** Verfahren gemäß Anspruch 1, dadurch gekennzeichnet, daß man Verbindungen allyliert, bei denen

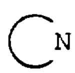

sich von Pyridinen, Chinolinen oder Isochinolinen ableitet, die noch durch $C_1$- bis $C_4$-Alkyl substituiert sein können.

**3.** Verfahren gemäß Anspruch 1, dadurch gekennzeichnet, daß man Pyridin allyliert.

**4.** Verfahren gemäß Anspruch 1, dadurch gekennzeichnet, daß man als Verbindungen der Formel III Allyl- oder α-, β-, γ-Methallyl-halogenid verwendet.

**5.** Verfahren gemäß Anspruch 1, dadurch gekennzeichnet, daß $X^\ominus$ Chlorid oder Bromid ist.

**6.** Verfahren gemäß Anspruch 1, dadurch gekennzeichnet, daß man die Umsetzung gegebenenfalls in Gegenwart

eines Emulgators und/oder eines wasserlöslichen organischen Lösungsmittels durchführt.

7. Verfahren gemäß Anspruch 1, dadurch gekennzeichnet, daß man die Umsetzung mit stöchiometrischen Mengen der Verbindungen II und III vornimmt.

8. Verfahren gemäß Anspruch 1, dadurch gekennzeichnet, daß man nicht umgesetzte Teile der Verbindungen II und/oder III mit Hilfe einer Wasserdampfdestillation oder durch Durchleiten eines inerten Gases entfernt, wobei ein pH-Wert von 4 bis 8, vorzugsweise 5 bis 6 eingehalten wird.

9. Verwendung der gemäß Anspruch 1 hergestellten Verbindungen als Glanzbildner bei der Herstellung vernickelter Formteile durch galvanisches Abscheiden von Nickel aus wäßrig-sauren Lösungen.

## Claims

1. A process for the preparation of an N-allyl compound of the formula I

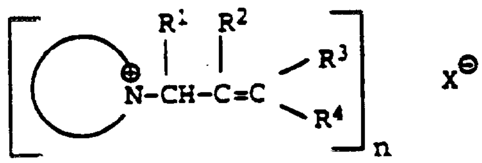

I,

where 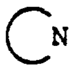 is an N-containing aromatic heterocyclic structure,

| | |
|---|---|
| $R^1$, $R^3$ and $R^4$, | independently of one another, are each hydrogen or $C_1$-$C_4$-alkyl, |
| $R^2$ | is hydrogen or methyl, |
| n | is 1, 2, 3 or 4 and |
| $X^{\ominus}$ | is a solubilizing anion |

wherein a compound of the formula II

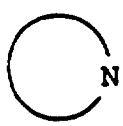

II,

is reacted with a compound of the formula III

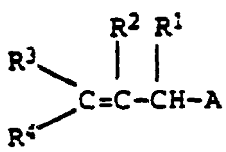

III,

where A is a radical which can be eliminated as an anion, in an aqueous medium.

2. A process as claimed in claim 1, wherein a compound in which

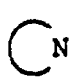

is derived from a pyridine, quinoline or isoquinoline which may furthermore be substituted by $C_1$-$C_4$-alkyl is allylated.

3. A process as claimed in claim 1, wherein pyridine is allylated.

4. A process as claimed in claim 1, wherein an allyl or α-, β- or γ-methallyl halide is used as the compound of the formula III.

5. A process as claimed in claim 1, wherein $X^{\ominus}$ is chloride or bromide.

6. A process as claimed in claim 1, wherein the reaction is carried out in the presence or absence of an emulsifier or of a water-soluble organic solvent.

7. A process as claimed in claim 1, wherein the reaction is carried out using stoichiometric amounts of the compounds II and III.

8. A process as claimed in claim 1, wherein unconverted parts of the compound II or III or of the compounds II and III are removed with the aid of steam distillation or by passing through an inert gas, a pH of from 4 to 8, preferably from 5 to 6, being maintained.

9. Use of a compound prepared as claimed in claim 1 as a brightener in the production of nickel-plated shaped articles by electrodeposition of nickel from an aqueous acidic solution.

**Revendications**

1. Procédé de préparation de composés N-allyliques de la formule générale I

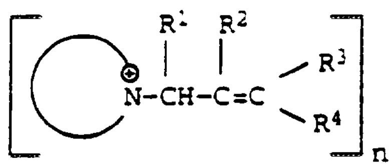 $X^{\ominus}$ I,

dans laquelle 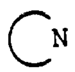 représente un hétérocycle aromatique contenant de l'azote,

| | |
|---|---|
| $R^1$, $R^3$ et $R^4$ | représentent, chacun indépendamment l'un de l'autre, un atome d'hydrogène ou un radical alkyle en $C_1$ à $C_4$, |
| $R^2$ | représente un atome d'hydrogène ou le radical méthyle, |
| n | est égal à 1, 2, 3 ou 4 et |
| $X^{\ominus}$ | représente un anion hydrosolubilisant, |

caractérisé en ce que l'on fait réagir des composés de la formule II

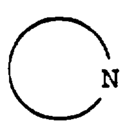 II,

avec des composés de la formule III

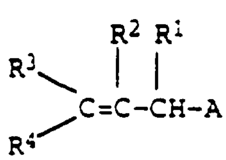 III,

dans laquelle A représente un radical séparable sous forme d'anion, dans un milieu aqueux.

2. Procédé suivant la revendication 1, caractérisé en ce que l'on allyle des composés dans lesquels

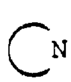

dérive de pyridines, de quinoléines, ou d'isoquinoléines, qui peuvent encore être substituées par des radicaux alkyle

en $C_1$ à $C_4$.

**3.** Procédé suivant la revendication 1, caractérisé en ce que l'on allyle de la pyridine.

**4.** Procédé suivant la revendication 1, caractérisé en ce que l'on utilise, à titre de composés de la formule III, un halogénure d'allyle ou d'α-, β-, γ-méthallyle.

**5.** Procédé suivant la revendication 1, caractérisé en ce que $X^{\ominus}$ représente un radical chlorure ou bromure.

**6.** Procédé suivant la revendication 1, caractérisé en ce que l'on opère la réaction éventuellement en présence d'un émulsif et/ou d'un solvant organique soluble dans l'eau.

**7.** Procédé suivant la revendication 1, caractérisé en ce que l'on entreprend la réaction avec des proportions stoechiométriques des composés II et III.

**8.** Procédé suivant la revendication 1, caractérisé en ce que l'on élimine les parties non converties des composés II et/ou III à l'aide d'une distillation à entraînement par la vapeur d'eau, ou par le passage d'un gaz inerte, où l'on maintient une valeur de pH de 4 à 8, de préférence de 5 à 6.

**9.** Utilisation des composés préparés suivant la revendication 1 à titre d'agents de brillantage lors de la fabrication d'articles moulés nickelés par le dépôt galvanique de nickel à partir de solutions aqueuses-acides.